# EUROPEAN PATENT APPLICATION

(11) **EP 2 801 384 A1**
(43) Date of publication of application: **12.11.2014**
(21) Application number: 13167362.6
(22) Date of filing: 10.05.2013
(51) Int. Cl.: A61M 16/04

(54) **Tube for endobronchial intubation**

(71) Applicant: Universität Zürich, 8006 Zürich (CH)
(72) Inventor: Weiss, Markus, 8053 Zürich (CH)
(74) Representative: Schulz, Ben Jesko

(57) **Abstract**

The invention relates to tube (1) for endobronchial intubation, comprising an endotracheal portion (100) being designed to be inserted into the trachea (10) of a person, particularly a child, the endotracheal portion (100) being in fluid communication with an inlet (O) at a proximal end (10) of the tube (1) for feeding a ventilation gas (G), particularly air, into the tube (1), an end region (101) of the endotracheal portion (100) opposing the proximal end (2), said end region (101) comprising a first opening (102) for discharging said ventilation gas (G) in one of the main bronchi (11) and a second opening (103) for discharging ventilation gas (G) in the other main bronchus (12), an endobronchial portion (200) being designed to be inserted into said other main bronchus (12), wherein the endobronchial portion (200) is connected to said end region (101) and is in fluid communication with said second opening (103) such that said ventilation gas (G) can be discharged through the second opening (103) into the endobronchial portion (200), wherein the endobronchial portion (200) further comprises an outlet (O') at a distal end (3) of the tube (1), so that said ventilation gas (G) can be discharged through said outlet (O') into said other main bronchus (12), and wherein particularly said endotracheal portion (100) and said endobronchial portion (200) of the tube (1) surround a single lumen (I) for the passage of said ventilation gas (G) through the tube (1) from said inlet (O) to said outlet (O'). According to the invention, the tube (1) comprises a particularly rotatable closure element (300; 400; 500; 600) for closing and opening said first and/or second opening (102, 103).

## Description

The invention relates to a tube for endobronchial intubation of a person, particularly for artificial ventilation of said person and/or single lung ventilation of said person.

A separation of the ventilation of the airway of the right or the left human lung is desired or even indicated in case of a single lung ventilation during a thorascopy, a single lung ventilation during thoracotomy, a single lung ventilation for immobilization of a right or a left lung (e.g. in case of a bleeding or fistula), for preventing of aspiration from the left lung into the right lung and vice versa (caverns), and for suction of the right or left lung (intensive care).

The selective ventilation of left and/or right lung can be performed by conducting an endotracheal intubation using a conventional single lumen ventilation tube, wherein the right or left main bronchus is blocked/opened by means of a blocking balloon. The blocking balloon may be inserted into the corresponding main bronchus outside the ventilation tube (extraluminal). Alternatively, a Fogarty catheter having a blocking balloon may be inserted into the corresponding main bronchus through the lumen of the ventilation tube in the trachea (Arndt endobronchial blocker system), i.e., intraluminal. Further, a Fogarty catheter having a blocking balloon may also be inserted into the corresponding main bronchus through the wall of the ventilation tube in the trachea (univent tube), i.e., intramural. However, disadvantageously, such blocking balloons may dislodge from the corresponding main bronchus into the opposite main bronchus or into the trachea during surgery and may be difficult or even impossible to become repositioned during surgery. A further disadvantage of blocking balloons is their low volume - high pressure characteristics leading, when sufficiently inflated to block the main bronchial lumen, to high pressures exerted to the bronchial mucosa. This may result in necrosis, ulceration, granulation and stenosis of the corresponding bronchus.

Most frequently, double lumen tubes are used, since these tubes are easy to position and reliable. However, disadvantageously, such tubes comprise a relatively small inner diameter causing a high resistance and demanding for specialized optics for conducting thoracic anesthesia. A further disadvantage is due to relatively large outer diameter and stiffness of the tube (due to the double lumen) which may cause damage to the larynx or may cause damage (even perforation) to the trachea as well as problems concerning intubation.

Since double lumen tubes are only available and applicable up to a certain size in pediatric anesthesia (OD-ID-ratio), bronchus blockers are employed today for children under the age of 10 (e.g. extramural, intraluminal or intramural).

Maneuvering and positioning of bronchus blockers is not always easy, especially in case of secretions and blood it is often difficult. Frequently, these blocker balloons tend to dislocate.

Extramural bronchus blockers may harm the larynx. Intramural bronchus blockers reduce the inner diameter of the tube, so that small inner diameters are employed in case of tall children. Further, intraluminal bronchus blockers reduce the inner cross-sectional area, the lumen for the bronchoscopy, and also interfere with suction catheters and fiberoptic endoscopes inside the lumen.

Generally, blocker catheters are equipped with a low-volume-high-pressure cuff and thus have the potential to harm the bronchi (necrosis) or to seal inherently bad when inflated.

Thus, the problem underlying the present invention is to provide for a tube, that reduces the risk of damaging the larynx, can be well controlled endoscopically, allows for atraumatic and easy insertion, comprises a high stability, and can particularly also be inserted nasally and / or via a tracheostomy into the trachea.

This problem is solved by a tube for endobronchial intubation having the features of claim 1.

According thereto, the tube comprises an endotracheal portion being designed to be inserted into the trachea, the endotracheal portion communicating with an inlet at a proximal end of the tube for feeding a ventilation gas, particularly air, into the tube; an end region of the endotracheal portion opposing the proximal end, said end region comprising a first opening for discharging said ventilation gas in one of the main bronchi (e.g. right main bronchus) in an open state of the first opening and a second opening for discharging gas in the other main bronchus (e.g. left main bronchus) in an open state of the second opening; an endobronchial portion being designed to be inserted into said other main bronchus, wherein the endobronchial portion is connected to said end region and communicates with said second opening such that said ventilation gas can be discharged through the second opening into the endobronchial portion (in an open state of the second opening), wherein the endobronchial portion further comprises an outlet (opposing the second opening) at a distal end of the tube, so that said ventilation gas (e.g. oxygen or air) can be discharged through said outlet into said other main bronchus, wherein particularly said endotracheal portion and said endobronchial portion merely surround a single lumen (or cavity) for the passage of said ventilation gas from said inlet to said outlet of the tube, and wherein - according to the invention - the tube comprises an element for closing and opening said first and/or second opening.

According to a preferred embodiment of the present invention, the closure element is designed to be rotatable.

Particularly, the closure element can be rotated from a first into a second position and vice versa. Preferably, in the first position, the closure element closes the second opening by covering it and opens the first opening by uncovering it. Further, in the second position, the closure element preferably closes the first opening by covering it and opens the second opening by uncovering it.

Particularly, the closure element can also be rotated into a third position. In said the third position the closure element preferably opens the first as well as the second opening at the same time by uncovering them.

Particularly, the closure element comprises an aperture (which may comprise an elongated shape), wherein in the first position of the closure element said aperture communicates with the first opening, i.e., ventilation gas can flow through said aperture and thereafter through the first opening, and wherein in the second position of the closure element said aperture communicates with the second opening (ventilation gas can flow through the aperture and thereafter through the second opening), and wherein particularly in said third position the aperture communicates with both the first and the second opening.

Particularly, in addition or alternatively, the closure element comprises a covering region, wherein in the first position of the closure element (or covering region) the covering region merely covers the second opening for blocking passage of ventilation gas through the second opening, i.e., ventilation gas can only flow through the first opening, and wherein in the second position of the closure element said covering region merely covers the first opening for blocking passage of ventilation gas through the first opening such that ventilation gas can flow through the second opening, and wherein particularly in said third position the covering region leaves open both the first and the second opening, so that ventilation gas can flow through both of the openings.

Particularly, the closure element may be formed as a tapered region or may comprise such a tapered region (e.g. a cone, wherein the tip of the cone may be rounded), in which tapered region said aperture is preferably formed (e.g. the aperture is formed in a mantle of the cone/tapered region along its periphery).

According to a further embodiment, the closure element is designed to (particularly merely) close and open said first opening, wherein the closure element is designed to be rotated between a first and a second position, wherein in the first position, the closure element opens the first opening by uncovering it, and wherein in the second position, the closure element closes the first opening by covering it.

Particularly, the closure element comprises an aperture, wherein in the first position of the closure element said aperture lies on top of the first opening, and wherein in the second position of the closure element said aperture lies on top of a wall region of said end region, and/or the closure element comprises a covering region wherein in the first position of the closure element the covering region lies on top of a wall region of said end portion and leaves the first opening open, and wherein in the second position of the closure element said covering region covers the first opening for blocking passage of ventilation gas through the first opening.

Particularly, the closure element comprises a cylindrical region or is formed as a cylindrical region or cylinder, in which cylindrical region said aperture is formed.

Preferably, for opening and closing the second opening, the tube comprises a further closure element in the form of an inflatable balloon that is designed to close the second opening in an inflated state, and to leave the second opening open in a non-inflated state. Preferably, the further closure element is arranged in said single lumen or cavity of the tube, preferably in a region of said single lumen or cavity delimited by the endobronchial portion of the tube.

It is also possible that the further closure element (e.g. inflatable balloon) is fastened to the closure element. In this respect, the inflatable balloon may be directly fastened to the closure element or via a conduit (e.g. inflation line) of the inflatable balloon that may extend along or inside the closure element (particularly along or inside said cylindrical region), wherein such an inflation line may also protrude from the closure element into the endobronchial portion of the tube (together with the further closure element connected to said inflation line), so that the inflatable element can be inflated in the endobronchial portion in order to close the second opening (or deflated in order to open the second opening).

Generally, the endobronchial portion of the tube preferably extends along a longitudinal axis.

Preferably, in case of the above embodiments, the closure element, particularly the tapered region or the cylindrical region, is designed to be rotated about the longitudinal axis of the endobronchial portion in order to open/close the two openings or the first opening.

Preferably, in case the above embodiments, the closure element, particularly the tapered region or the cylindrical region, is designed to rest with an outer side against an inner side of the end region of the endotracheal portion of the tube, particularly with a positive fit, wherein said inner side faces the (e.g. single) lumen or cavity of the tube, i.e., the inner space defined by the tube serving for the passage of ventilation gas (e.g. air) through the tube.

Preferably, in case of the above embodiments, the closure element is formed as an inner tube inserted into the endotracheal portion in form fitting manner, wherein said inner tube protrudes out of a further end region of the endotracheal portion with a first end section forming said proximal end of the tube and delimiting said inlet, and wherein particularly said tapered region or cylindrical region forms an opposing second end section of the inner tube.

Preferably, in case of the above embodiments, said first end section of the closure element is designed to be rotated manually in order to rotate the closure element back and forth between the individual positions, i.e., the first end section of the closure element (inner tube) serves as a handle.

According to a further embodiment of the present invention, the tube comprises an arresting means for arresting the closure element (inner tube) in the respective first, second, and/or third position. Preferably, the arresting means is designed to arrest the closure element in said positions by fixing the first end section of the closure element to said further end region of the endotracheal portion of the tube opposing said end region housing the tapered/cylindrical region of the closure element (inner tube).

According to a further embodiment of the present invention, the tube comprises an indicating means connected to the first end section of the closure element being designed to indicate in which of said positions the closure element currently resides. Thus, a physician or medical personnel can easily determine the actual position of the closure element.

The part of the inner tube connecting to said tapered region of the inner tube (closure element) may also be formed as a fenestrated inner tube section, i.e., must not form a continuous wall.

In an alternative embodiment of the present invention, an elongated rod is provided instead, which rod is connected or can be releasably connected to the closure element, particularly to said tapered region of the closure element, so as to rotate the closure element by rotating said rod, which rod preferably protrudes out of the inlet of the tube when connected to the closure element. The rod may comprise a head that may engage with a socket (e.g. a hexagon socket) of the closure element or vice versa for releasably connecting the rod to the closure element. Rotation of the closure element may also be accomplished by two rods connecting to e.g. opposing sides of the closure element.

In a further alternative embodiment of the present invention, at least one cable, preferably two cables, may be provided, which are coupled to the closure element, particularly to the tapered region of the latter, so as to rotate the closure element by pulling the at least one cable, wherein said cable(s) preferably extend in a recess formed in a wall of the endotracheal portion and protrude out of the latter so that they can be operated manually or connected to an actuation means for actuating said cable(s).

Particularly, in case of the above embodiments, the tapered region or cylindrical region of the closure element may comprise guiding grooves or guiding projections, wherein particularly such guiding grooves are preferably designed to engage with corresponding projections formed on an inner side of the end region of the endotracheal portion of the tube, and wherein particularly in case said tapered region or cylindrical region comprises guiding projections, the latter are designed to engage with corresponding grooves formed in said inner side of said end region of the endotracheal portion of the tube.

According to a further embodiment of the present invention, the closure element is designed to (particularly merely) open and close said first opening, wherein the closure element is designed as a sliding element that is designed to be displaced between a first and a second position, particularly along the longitudinal axis of the endotracheal portion of the tube, wherein in the first position the closure element leaves the first opening open, and wherein in the second position the closure element covers the first opening. Preferably, the closure element may have a tubular shape, and is preferably arranged with a positive fit in the lumen or cavity of the tube in the end region of the endotracheal portion.

Preferably, the closure element is arranged in said single lumen (cavity) of the tube and is preferably connected to an elongated member (e.g. rod) extending inside said lumen along the longitudinal axis of the endotracheal portion, wherein preferably an end region of said elongated member is connected to a handle (e.g. slider) arranged in the region of the proximal end of the tube that can be slid back and forth along the longitudinal axis of the endotracheal portion taking along the elongated member and the closure element connected thereto, such that the closure element can be displaced back and forth between the first and second position manually by means of said handle.

Preferably, for opening and closing the second opening, the tube comprises a further closure element in the form of an inflatable balloon that is designed to close or block the second opening for preventing the passage of ventilation gas through the second opening in an inflated state, and to leave the second opening open in a non-inflated state so that ventilation gas can pass the second opening. Preferably, said further closure element is fastened to the closure element for closing the first opening, such that the further closure element can be displaced together with the closure element, Preferably, the inflatable balloon is encompassed by the (e.g. tubular) closure element.

Further, in the first position, the closure element is preferably arranged beyond the first opening along the longitudinal axis of the endotracheal portion so that the further closure element may close the second opening but still allows for discharging ventilation gas flowing in the endotracheal portion through the first opening.

In yet another preferred embodiment of the present invention, the closure element being formed as a sliding element may be alternatively arranged in a separate cavity extending along the longitudinal axis of the endotracheal portion of the tube, which separate cavity is preferably formed in a wall of the endotracheal portion. In this case, the closure element is preferably formed as an elongated sliding element comprising an aperture at a first end, which, in the first position of the closure element is aligned with the first opening (or lies on top of it) such that ventilation gas can be discharged through the first opening. In the second position, said aperture is preferably arranged away from the first opening, so that the first opening is covered by the closure element and is thus closed (i.e. blocked for preventing the passage of ventilation gas). Preferably, in the second position, the closure element is inserted deeper into said separate cavity than in the first position, which particularly means that the aperture of the closure element is then displaced beyond the first opening. Hence a region of the sliding element adjacent to said aperture then closes the first opening by covering it. Further, said separate cavity preferably communicates with the first opening. As before, an opposing second end region of the sliding element is preferably connected to a handle (slider) arranged in the region of the proximal end of the tube that can be slid back and forth along the longitudinal axis of the endotracheal portion taking along the sliding element such that the sliding element (closure element) can be displaced back and forth between the first and second position manually by means of said handle.

Preferably, again a further closure element in the form of an inflatable balloon is provided that is designed to close the second opening in an inflated state, and to leave the second opening open in a non-inflated state. Preferably, the further closure element is arranged in said single lumen or cavity of the tube, preferably in region of said single lumen or cavity delimited by the endobronchial portion of the tube.

It is also possible that the further closure element (e.g. inflatable balloon) is fastened to the sliding element at the first end of the sliding element, particularly via a conduit (e.g. inflation line) of the inflatable balloon that may extend along or inside the sliding element, wherein said inflation line may protrude from the sliding element through a channel that connects said cavity with said single lumen of the tube in the region of the endobronchial portion of the tube so that the inflatable balloon can be inflated in the endobronchial portion in order to close the second opening.

Generally, in all embodiments, the tube preferably comprises an expandable endotracheal cuff encompassing the endotracheal portion of the tube, which endotracheal cuff is preferably designed to block flow of ventilation gas (e.g. air) through the trachea in an expanded state of the endotracheal cuff.

Further, in all embodiments, the tube preferably comprises an expandable endobronchial cuff encompassing the endobronchial portion of the tube, which endobronchial cuff is preferably designed to block flow of ventilation gas (e.g. air) through said other main bronchus in an expanded state of the endobronchial cuff. Furthermore, in all embodiments, the endobronchial portion is preferably connected to the endotracheal portion of the tube at an obtuse angle.

Further, in all embodiments, the endobronchial portion of the tube may be designed for insertion into the right main bronchus, wherein the endobronchial portion preferably comprises a further outlet formed in the endobronchial cuff or in a section of the endobronchial portion between said cuff and the distal end of the tube, wherein said further outlet is designed for discharging ventilation gas into the right upper lobe of the lung.

Further, in all embodiments, the tube preferably comprises in a transition region between the end region of the endotracheal portion and the endobronchial portion a protrusion (e.g. domical uplift) which is designed to prevent a further insertion of the tube or endobronchial portion into the respective main bronchus, thus particularly ensuring an optimal position of the whole tube, particularly of the outlet of the tube with respect to the respective main bronchus.

Preferably, in all embodiments, the endotracheal and endobronchial portion of the tube consists of at least one of the following materials: polyvinyl chloride and/or polyurethane. Other materials may also be used.

Preferably, in all embodiments, the closure element (e.g. inner tube) preferably consists of silicone. Other materials may also be used.

Further features and advantages of the invention shall be described by means of detailed descriptions of embodiments with reference to the Figures, wherein
- Fig. 1: shows a schematical cross-sectional view of a tube according to the invention having a closure element residing in a first position for opening a first opening to the right main bronchus and closing a second opening of the tube to the left main bronchus;
- Fig. 2: shows a schematical cross-sectional view of the tube according to Fig. 1, wherein the closure element resides in a second position for closing said first opening and for opening said second opening of the tube;
- Fig. 3: shows a schematical cross-sectional view of the tube according to Fig. 1, wherein the closure element resides in a third position for opening said first as well as said second opening;
- Fig. 4: shows a schematical view of an end region of an endotracheal portion of the tube comprising a first and a second opening for ventilation of the two main bronchi, as well as a schematical view of a region of the closure element for the openings comprising an aperture;
- Fig. 5: shows schematical views of said region of the closure element being in the first position, the second position, and in the third position;
- Fig. 6: shows schematical views of a covering region of a closure element being in the first position, the second position, and in the third position;
- Fig. 7: shows a perspective view of a tube according to the invention having a rotatable inner tube as a closure element;
- Fig. 8: shows a perspective view of a detail of the tube according to Fig. 7; and
- Fig. 9-11: show cross-sectional views of a part of the tube according to Fig. 7 showing the closure element in its first, second and third position, respectively;
- Fig. 12: shows a cross-sectional view of the closure element having guiding grooves and projections for precise adjustment of the position of the closure element;
- Fig. 13: shows a cross-sectional view of a further embodiment of the tube according to the invention, wherein the first opening is open, and the second opening is closed by a further closure element in the form of an inflatable balloon;
- Fig. 14: shows a cross-sectional view of the embodiment of Fig. 13, wherein the first opening is closed by the closure element, and the further closure element is in a non-inflated state leaving the second opening open;
- Fig. 15: shows a detail of the embodiment shown in Figs. 13 and 14;
- Fig. 16: shows a cross-sectional view of a further embodiment of the tube according to the invention, wherein the closure element is formed as a sliding element leaving the first opening open, and wherein a further closure element formed as an inflatable balloon being fastened to the closure element closes the second opening in an inflated state;
- Fig. 17: shows a cross-sectional view of the embodiment of Fig. 16, wherein the closure element closes the first opening, and wherein the further closure element is in a non-inflated state leaving the second opening open;
- Fig. 18: shows a detail of the embodiment shown in Figs. 16 and 17;
- Fig. 19: shows a detail of yet another embodiment of the tube according to the invention, wherein the closure element is formed as a sliding element housed in a separate cavity formed in a wall of the endotracheal portion of the tube;
- Fig. 20: shows a closure element in the form of an elongated sliding element of the embodiment shown in Fig. 19;
- Fig. 21: shows a cross-sectional view of the embodiment shown in Fig. 19 with an open first opening and an open second opening;
- Fig. 22: shows a cross-sectional view of the embodiment of Figs. 19 to 21, wherein the first opening is open and the second opening is closed by a further closure element in the form of an inflatable balloon;
- Fig. 23: shows a cross-sectional view of the embodiment of Figs. 19 to 22, wherein the first opening is closed by the closure element, and wherein the further closure element is in a non-inflated state leaving the second opening open; and
- Fig. 24: shows a detail of the embodiment shown in Figs. 19 to 23.

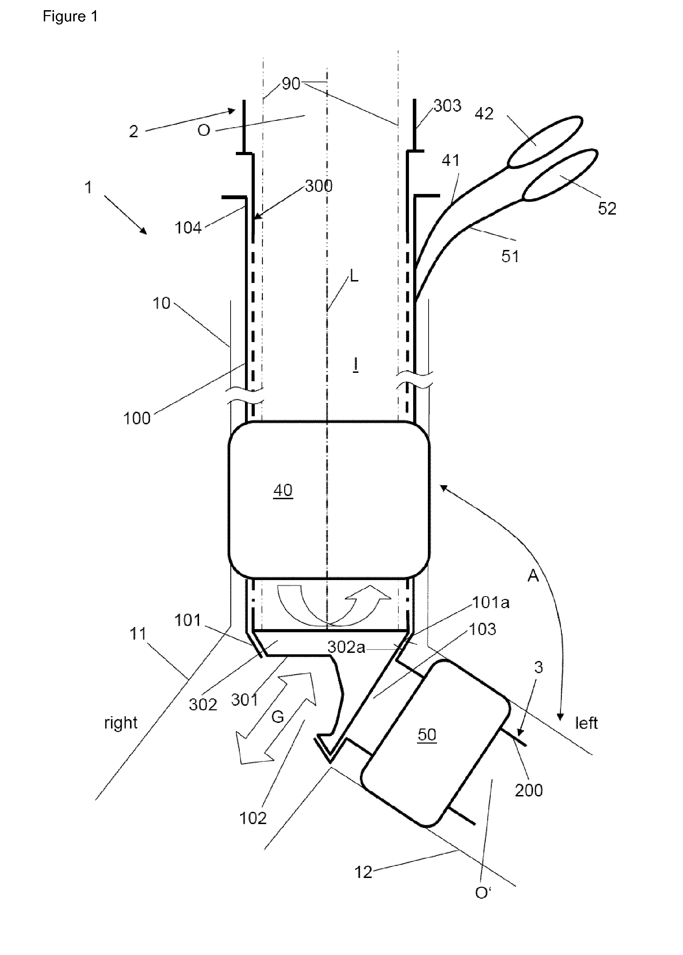

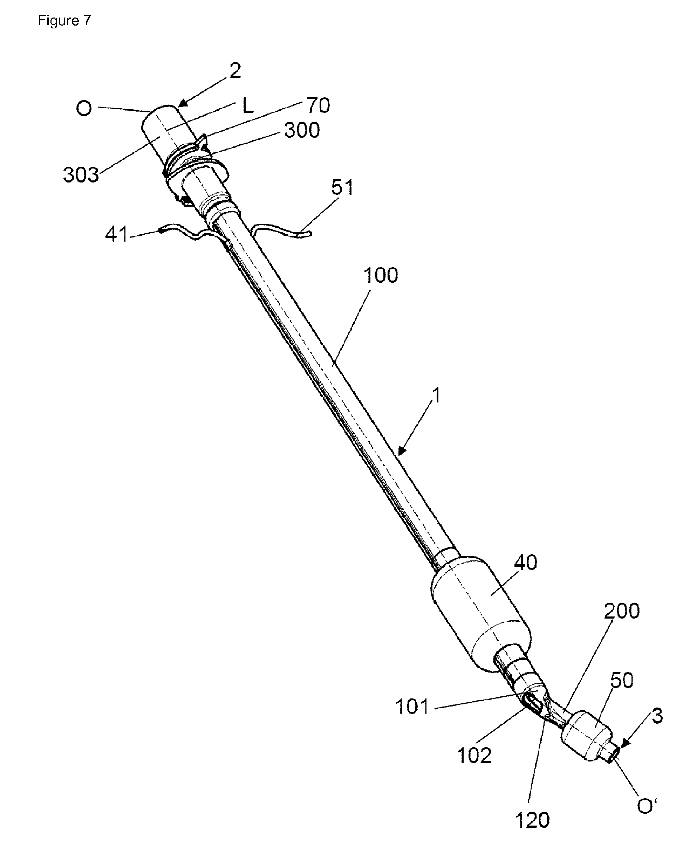

Figure 1 (cf. also Fig. 7) shows a schematic view of a tube 1 for endobronchial intubation of a person, particularly of a child.

The tube 1 comprises a tubular endotracheal portion 100 extending along a longitudinal axis L, which endotracheal portion 100 is designed to be inserted (at least in sections) into the trachea 10 of said person. The endotracheal portion 100 connects to an inlet O of the tube 1 at a proximal end 2 of the tube 1 for feeding a ventilation gas G, normally oxygen or air, into the tube 1, for ventilating either the right, left, or right and left lung of said person.

The endotracheal portion 100 further comprises an end region 101 opposing said proximal end 2 of the tube 1 along the longitudinal axis L, wherein said end region 101 comprises a first opening 102 for discharging said ventilation gas G fed into the endotracheal portion 100 via inlet O in one of the main bronchi 11, e.g. the right bronchus 11 as shown in Fig. 1, and a second opening 103 for discharging said ventilation gas G in the other main bronchus 12, e.g. the left bronchus 12 according to Fig. 1.

Here, the second opening 103 is in fluid communication with an endobronchial portion 200 of the tube 1, which is designed to be inserted into said other main bronchus 12, e.g. the left bronchus 12, and connects to said end region 101 of the endotracheal portion 100 under an obtuse angle A. Thus, said ventilation gas G can be discharged through the second opening 103 into the endobronchial portion 200, when said second opening 103 is open for the passage of ventilation gas G.

The endobronchial portion 200 further comprises an outlet O' at a distal end 3 of the tube 1, so that ventilation gas G that has been fed into the endobronchial portion 200 via said second opening 103 can be discharged through said outlet O' into said other main bronchus (e.g. left bronchus) 12.

The endotracheal portion 100 and the endobronchial portion 200 surround merely a single lumen or cavity l for the passage of said ventilation gas G through the tube 1 from said inlet O to said outlet O', so that the outer diameter of the tube 1, particularly the endotracheal portion 100 can be sufficiently small for reducing the risk of damage to the airways, particularly the larynx.

In order to be able to separate the right lung from the left lung or also to permit ventilation of both lungs at the same time, the tube 1 comprises a rotatable closure element 300 for closing and opening said first and second opening 102, 103.

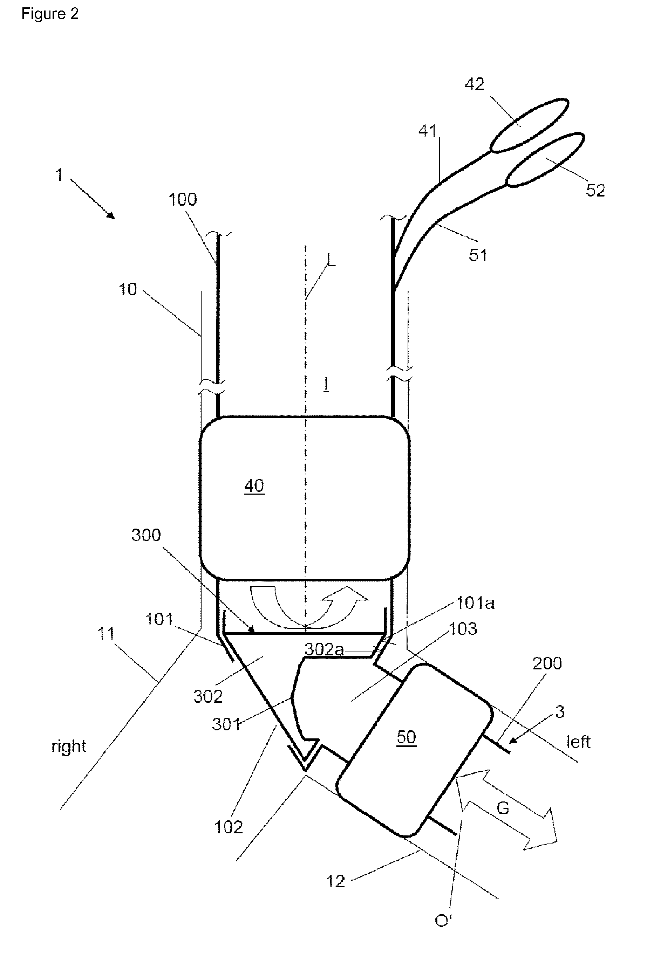

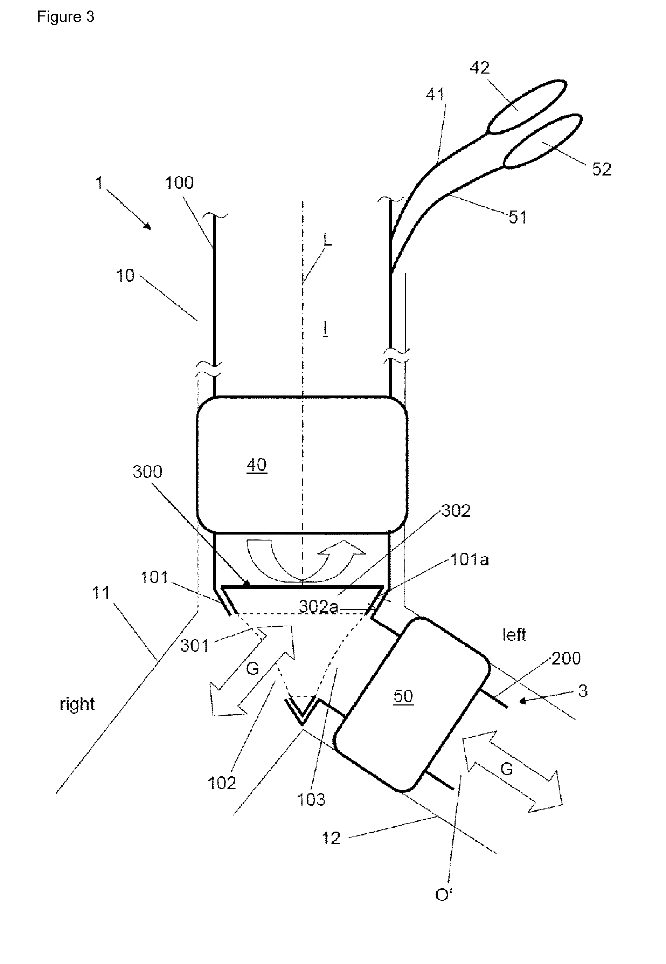

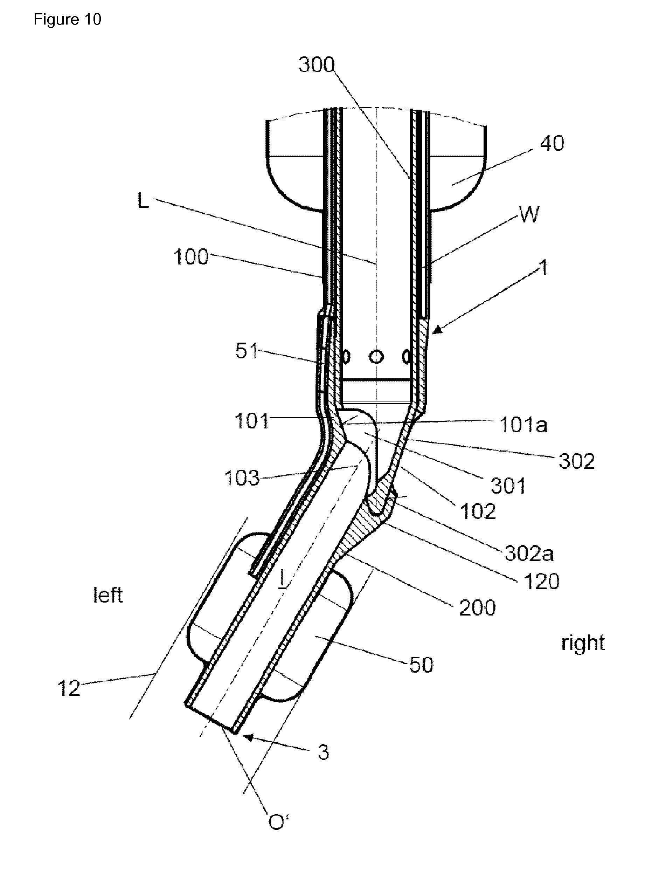

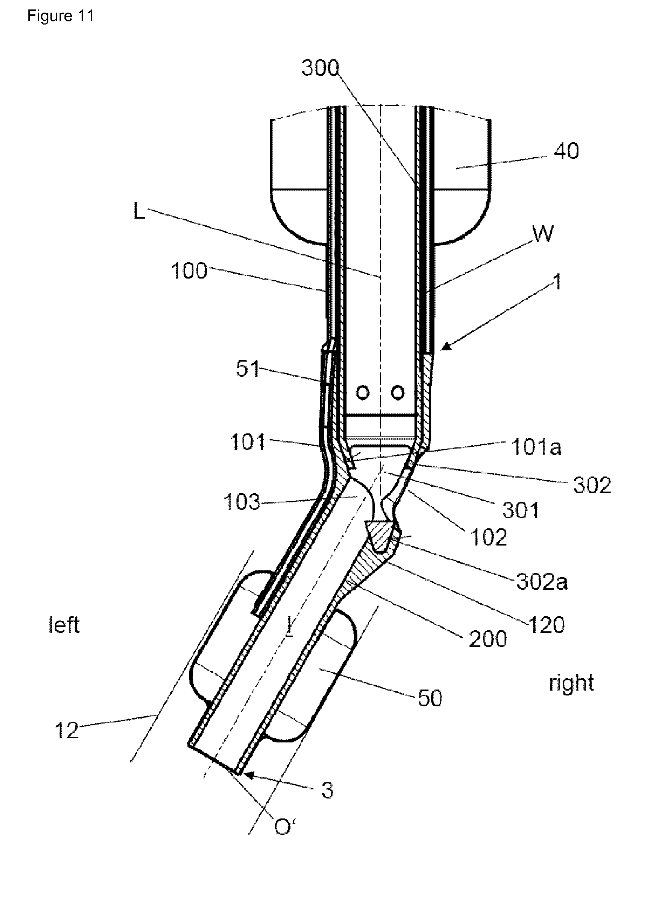

As shown in Figs. 1 to 6, as well as Figs. 9-11 the closure element 300 is designed to be rotated about the longitudinal axis L forming a rotation axis, wherein the closure element 300 can be arranged in a first position shown in Figs. 1, 9, 5 (upper part) and 6 (upper part), in a second position shown in Figs. 2, 10, 5 (middle part) and 6 (middle part), and in a third position shown in Figs. 2, 11, 5 (lower part) and 6 (lower part).

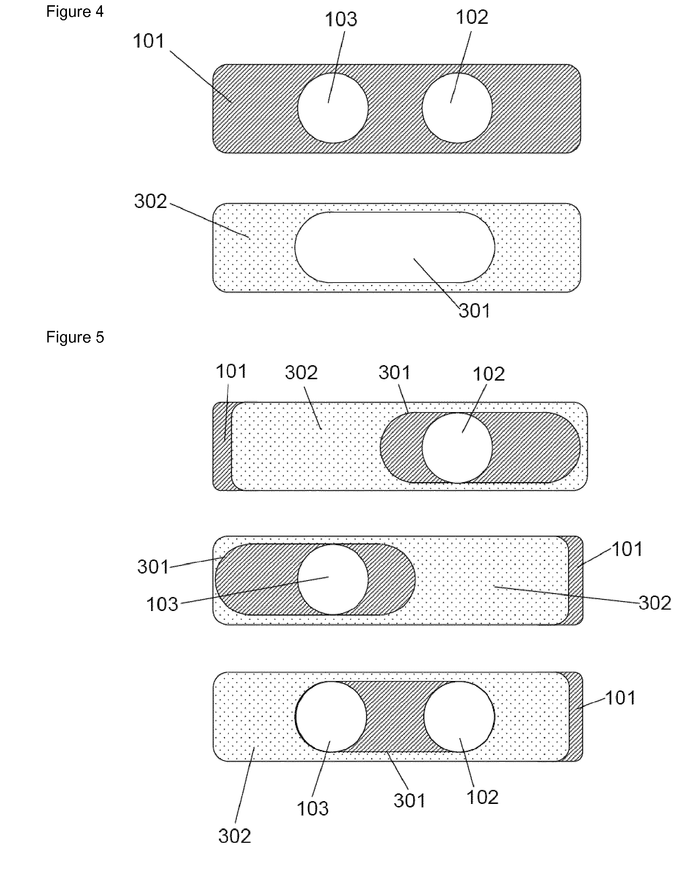

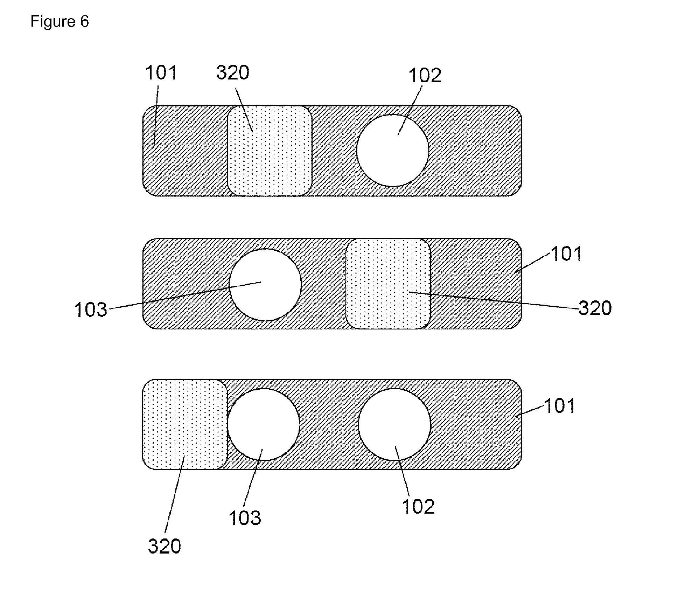

As schematically shown in Fig. 4 upper part, the end region 101 of the endotracheal portion 100 comprises the first opening 102 and the separately formed second opening 103. For closing and opening these openings 102, 103 the closure element 300, particularly a tapered region 302 of the closure element 300, comprises an aperture 301, which - when rotating the closure element 300 about said rotation axis - can be arranged in said first position on top of the first opening (cf. Fig. 5 upper part) such that the closure element (e.g. tapered region) 302 blocks the second opening 103, but leaves the first opening 102 open. In the second position, as shown in Fig. 5 (middle part), the closure element (e.g. tapered region) 302 blocks the first opening 102, but leaves open the second opening 103, whereas in the third position (cf. Fig. 5 lower part), the closure element (e.g. tapered region) 302 leaves both openings 102, 103 open, thus allowing ventilation of both lungs. Particularly, the closure element 300 may also have other shapes. In this respect, it is merely crucial that the closure element 300 is designed to leave either one of the openings 102, 103 open and close the other one, or leave both of them open, when rotated in the respective position. For instance, as shown schematically in Fig. 6 the closure element 300 may also comprise a covering region 320, wherein in the first position of the closure element 300 (or covering region 320) the covering region 320 merely covers the second opening 103 for blocking passage of ventilation gas G through the second opening 103 such that ventilation gas G can flow through the first opening 102, and wherein in the second position of the closure element 300 said covering region 320 merely covers the first opening 102 for blocking passage of ventilation gas G through the first opening 102 such that ventilation gas G can flow through the second opening 103. Further, in said third position the covering region 320 then leaves open both the first and the second opening 102, 103, so that ventilation gas G can flow through both of the openings 102, 103. As further shown in Figs. 1 to 3 and 9 to 11, as well as Figs. 13 to 24 the tube 1 according to the invention comprises an expandable endotracheal cuff 40, which encompasses the endotracheal portion 100 of the tube 1. Said cuff 40 may be expanded by inflating it with a gaseous or liquid medium via a conduit 41(also denoted as inflation line) connected e.g. to a pilot balloon 42 which may comprise a syringe connector for connecting to a syringe (for delivering or removing said medium), by means of which pilot balloon 42 and cuff 40 said medium can be instilled or drawn off said cuff 40. Said endotracheal cuff 40 is designed to block the flow of ventilation gas G through the trachea 10 in an expanded state of the endotracheal cuff 40 by circumferentially expanding and thereby pressing against the internal wall of the trachea 10.

In order to block said other main bronchus (e.g. left bronchus) 12 for preventing the passage of inflation gas (e.g. oxygen or air) G, the tube 1 further comprises an expandable endobronchial cuff 50 encompassing the endobronchial portion 200 of the tube 1. Again, the endobronchial cuff 50 may be expanded by inflating it with a fluid medium via a conduit 51 (inflation line) connected e.g. to a pilot balloon 52. Likewise, said endobronchial cuff 50 is designed to block flow of ventilation gas G through the other main bronchus (e.g. left bronchus) 12 in an expanded state of the endobronchial cuff 50 by circumferentially extending and thereby pressing against the internal wall of the other main bronchus (e.g. left bronchus) 12. The conduits (so-called inflation lines) 41, 51 may be formed in or attached to the circumferentially extending (e.g. tubular) wall of the endotracheal portion 100 of the tube 1.

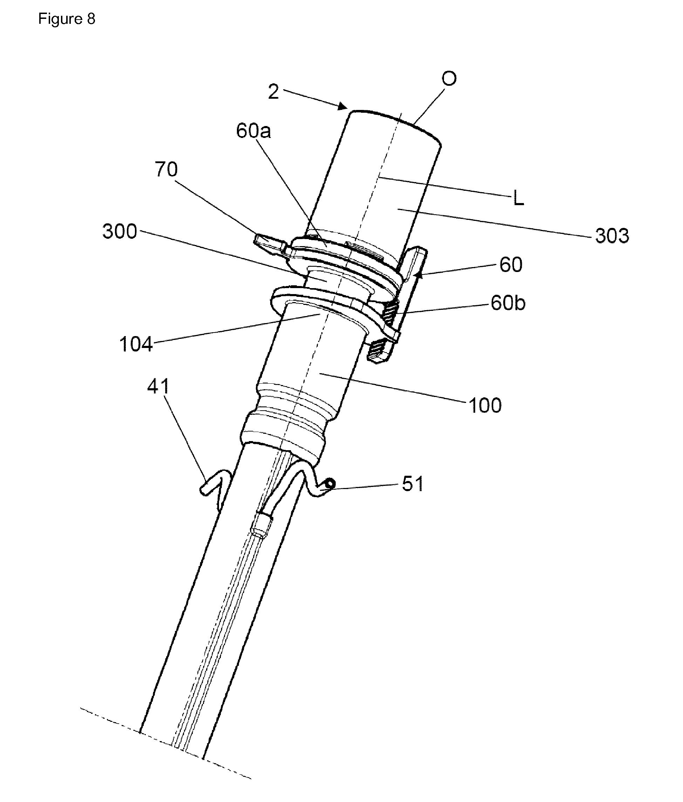

As indicated in Fig. 1 by the dashes lines, the tapered region 302 of the closure element 300 may be a second end section 302 of a closure element 300 in the form of an inner tube 300 inserted into the tubular endotracheal portion 100 of the tube 1, which inner tube 300 protrudes out of a further end region 104 of said endotracheal portion 100 opposing said end region 101 of the endotracheal portion 100 that houses the tapered region 302 of the inner tube (closing element) 300. Thus, the first end section 303 of the inner tube (closure element) 300 forms said proximal end 2 of the tube 1 which delimits said inlet O of the tube 1. Such an embodiment, where the closing element 300 is formed as an inner tube 300 having a continuous circumferentially extending tubular wall that is slideably arranged (particularly with a positive fit) in the endotracheal portion 100 of the tube 1 so that the lumen l is delimited by the inner tube 300 in the endotracheal portion 100 and the endobronchial portion 200 of the tube 1 is depicted in Figs. 7 to 11. Concerning the features of Figs. 7 to 11 it is also referred to the description of Figs. 1 to 6 above. Further, as can be seen from Figs. 7 and 8, the tube 1 may comprise an arresting means 60 for arresting the closure element (inner tube) 300 in its the first, second or third position 300 with respect to the surrounding endotracheal portion 100 of the tube 1.

The arresting means 60 is designed to clasp the first end section 303 of the inner tube 300 via a clamping part 60a and to engage via a toothed region 60b with the further end region 104 of the endotracheal portion 100, wherein the toothed region 60b engages such with said further end region 104 that the inner tube 300 is pressed into the endotracheal portion 100 of the tube 1 along the longitudinal axis L, so that the tapered region 302 is properly positioned in the end region 101 of the endotracheal portion 100 of the tube 1.

In order to change the position of the inner tube 300, said arresting means 60 is released from the further end region 104 of the endotracheal portion 100 and the inner tube 300 is slightly pulled out of the endotracheal portion 100, rotated into the desired position and then pushed back into the endotracheal portion 100, Then, the arresting means 60 is engaged again with said further end region 104 via said toothed region 60b, so that the inner tube 300 is arrested in its respective position as well as pre-tensioned with its tapered region 302 against said end region 101 of the endotracheal portion 100 of the tube 1.

Particularly, said pre-tensioning is necessary since the positive fit of the inner tube's 300 tapered region 302 in the end region 101 of the endotracheal portion 100 may be disturbed by bending the proximal (intraoral/pharyngeal) portion and/or endotracheal portion 100 (e.g. upon intubation).

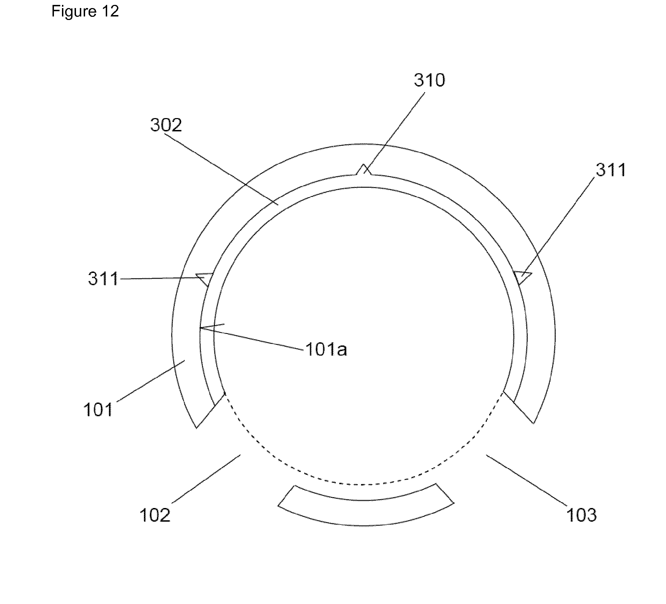

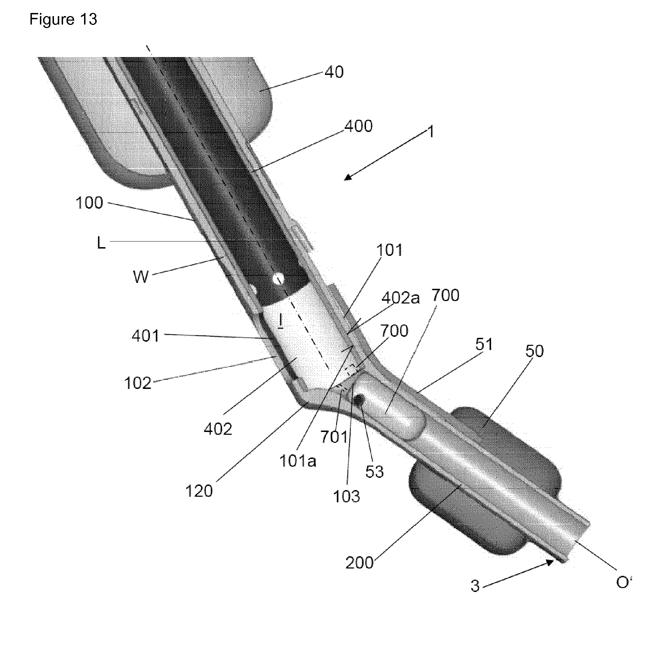

Particularly, in order to be able to secure the first, second or third position of the closure element 300 (or the first and second position of the closure element 400 according to Figs. 13 to 15) precisely, the tapered region 302 (or cylindrical region, see below) of the closure element 300 may comprise guiding projections 310, which are designed to engage with corresponding grooves 311 formed in said inner side 101 a of said end region 101 of the endotracheal portion 100 as shown in Fig. 12. Furthermore, an indicating means 70 protrudes from the first end section 303 of the inner tube 300 which preferably points in the direction of the aperture 301 of the closure element, so that the respective physician or medical personnel can see which main bronchi 11, 12 or 11 and 12 is/are ventilated. Further, the indicating means 70 may be used as a lever for rotating the inner tube/closure element 300.

In an alternative embodiment, as indicated in Fig. 1, the inner tube 300 may be fenestrated in a section connecting to said e.g. tapered region 302 of the inner tube (closure element) 300. Further, in case the closure element 300 is not formed as a (continuous) inner tube 300, the closure element 300 (e.g. tapered region 302) may be rotated by means of a single central (e.g. intraluminal) elongated rod 90 connected to the closure element 300 (or by means of two such rods 90 connected e.g. to opposing sides of the closure element 300), or alternatively by means of cable(s) 90, which may extend in the wall of the endotracheal portion 100 of the tube 1 towards its further end region 104.

Further, the first end section 303 of the inner tube (closure element) 300 particularly serves as a connecting part for connecting a breathing tube from the respirator to the inner ventilation tube 300.

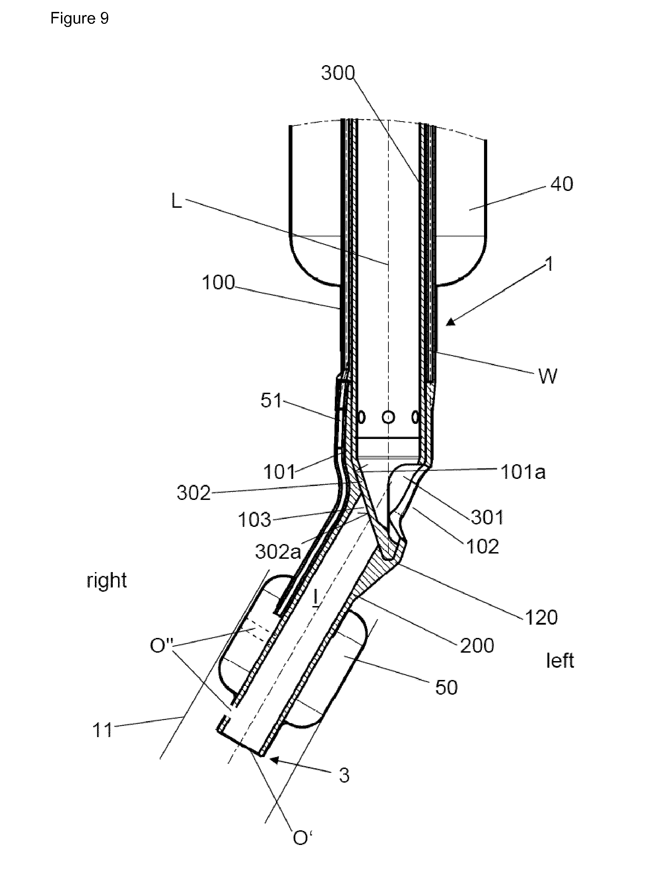

Further, as shown in Figs. 9 to 11 as well as Figs. 13, 14, 16, 17, 21 - 23 for instance, the tube 1 comprises in a transition region between the end region 101 of the endotracheal portion 100 and the endobronchial portion 200 a protrusion 120 (e.g. domical uplift) which is designed to prevent a further insertion of the tube 1 or endobronchial portion 100 into the respective main bronchus 11, 12, thus particularly ensuring an optimal position of the whole tube 1, particularly of the outlet O' of the tube 1 with respect to the respective main bronchus 11, 12.

Finally, the endobronchial portion 100 of the tube 1 may be designed for insertion into the right main bronchus 11 instead of the left main bronchus 12. Then, the endobronchial portion 100 may comprise an optional further outlet O" (indicated in Fig. 9 as an example) formed either in the endobronchial cuff 50 or in a section of the endobronchial portion 100 between said cuff 50 and the distal end 3 of the tube 1, wherein said further outlet O" is designed for discharging ventilation gas G into the right upper lobe of the lung.

Figs. 13 to 24 show further embodiments of the tube 1 according to the invention. Again, in these embodiments, as described with respect to Fig. 7 above, the respective tube 1 comprises a tubular endotracheal portion 100 extending along a longitudinal axis L, which endotracheal portion 100 is designed to be inserted into the trachea 10 of a person. The endotracheal portion 100 connects to an inlet O of the tube 1 at a proximal end 2 of the tube 1 for feeding a ventilation gas G, normally oxygen or air, into the tube 1, for ventilating either the right, left, or right and left lung of said person.

The endotracheal portion 100 further comprises an end region 101 opposing said proximal end 2 of the tube 1 along the longitudinal axis L, wherein said end region 101 comprises a first opening 102 for discharging said ventilation gas G fed into the endotracheal portion 100 via inlet O in one of the main bronchi 11, e.g. the right bronchus 11 as shown in Fig. 1, and a second opening 103 for discharging said ventilation gas G in the other main bronchus 12, e.g. the left bronchus 12 according to Fig. 1.

The second opening 103 is in fluid communication with an endobronchial portion 200 of the tube 1 (e.g. the second opening 103 may be defined as a transition region or section of the single lumen or cavity l of the tube 1 from the endotracheal portion 100 to the endobronchial portion 100), which is designed to be inserted into said other main bronchus 12, e.g. the left bronchus 12, and connects to said end region 101 of the endotracheal portion 100 under an obtuse angle A. Thus, said ventilation gas G can be discharged through the second opening 103 into the endobronchial portion 200, when said second opening 103 is open for the passage of ventilation gas G.

The endobronchial portion 200 further comprises an outlet O' at a distal end 3 of the tube 1, so that ventilation gas G that has been fed into the endobronchial portion 200 via said second opening 103 can be discharged through said outlet O' into said other main bronchus (e.g. left bronchus) 12.

Again, in Figs. 13 to 24, the endotracheal portion 100 and the endobronchial portion 200 surround merely a single lumen or cavity I for the passage of said ventilation gas G through the tube 1 from said inlet O to said outlet O', so that the outer diameter of the tube 1, particularly the endotracheal portion 100 can be sufficiently small for reducing the risk of damage to the airways, particularly the larynx.

In order to be able to separate the right lung from the left lung or also to permit ventilation of both lungs at the same time, the tube 1 according to Figs. 13 to 15 comprises a rotatable closure element 400 for closing and opening said first opening 102 and a further closure element 700 formed as an inflatable balloon 700, which is arranged in the endobronchial portion 200 adjacent to the second opening 103, wherein particularly the further closure element 700 is fastened to an inner side of the endobronchial portion 200. Thus, when the further closure element 700 is in an inflated state it closes or blocks the second opening 103, which means that ventilation gas G cannot flow through the endobronchial portion 200 and cannot be discharged through outlet O'. Note, that the further closure element 700 need not be arranged precisely in the second opening 103, but can be arranged in front or behind the second opening 103. Here, it is crucial that when the second opening 103 is closed, ventilation gas G cannot flow through the endobronchial portion 200 and cannot be discharged out of the outlet O'.

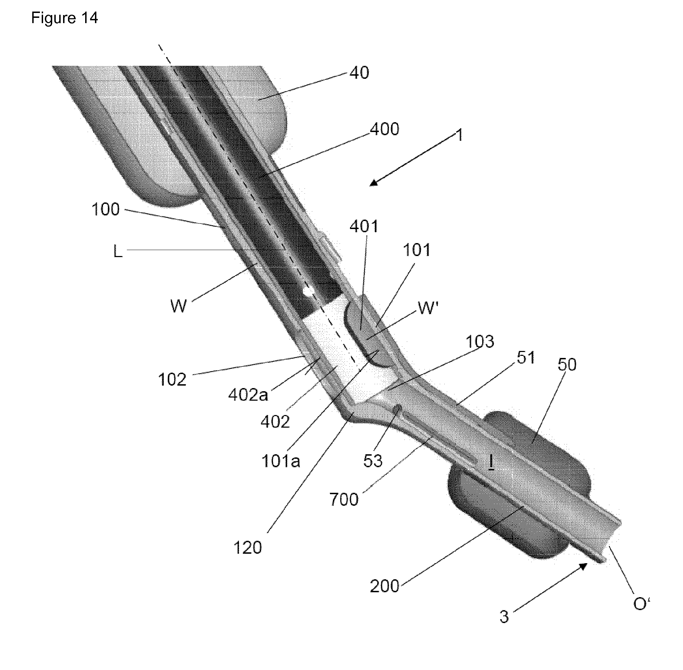

Alternatively, as indicated in Fig. 13 by dashed lines, it is also possible that the further closure element in form of the inflatable balloon 700 is fastened to an end region of the closure element 400. In this respect, the inflatable balloon 700 may be directly fastened to the closure element 400 or via a conduit (e.g. inflation line) 701 of the inflatable balloon 700 that may extend along or inside the closure element (particularly along or inside said cylindrical region) 400, wherein particularly the inflation line 701 protrudes from the closure element 400 into the endobronchial portion 200 of the tube 1 (together with the further closure element 700 connected to said inflation line), so that the inflatable balloon 700 can be inflated in the endobronchial portion 200 of the tube 1 in order to close the second opening 103 (or deflated so as to open the second opening 103). The closure element 400 is formed as an inner tube 400 having a continuous circumferentially extending tubular wall that is slideably arranged (particularly with a positive fit) in the endotracheal portion 100 of the tube 1 so that the lumen or cavity l is delimited by the inner tube 400 in the endotracheal portion 100 and the endobronchial portion 200 of the tube 1. Said inner tube 400 protrudes out of a further end region 104 of said endotracheal portion 100 with a first end section 403, so that said first end section 403 of the inner tube (closure element) 400 forms said proximal end 2 of the tube 1 which delimits said inlet O of the tube 1, wherein said further end region 104 of the endotracheal portion 100 opposes said end region 101 of the endotracheal portion 100. The closure element 400 further comprises a cylindrical region (second end section) 402 housed by the end region 101 of the endotracheal portion 100, wherein an outer side 402a of the cylindrical region 402 butts against in inner side 101 a of said end region 101 of the endotracheal portion 100. The cylindrical region 402 comprises an aperture 401, wherein the closure element or inner tube 400 can be rotated about the longitudinal axis L of the endotracheal portion 100 between a first position (shown in Fig. 13) in which said aperture 401 is aligned with the first opening 102, and a second position (shown in Fig. 14) in which said aperture 401 faces a wall portion W' of the end region 101 while the first opening 102 is blocked by a continuous portion (or covering region) of the cylindrical region 402. Thus, in the first position of the closure element 400 the first opening 102 is open and ventilation gas G can be discharged out of the first opening 102, while in the second position of the closure element 400 the first opening 102 is closed or blocked for preventing the passage of ventilation gas G.

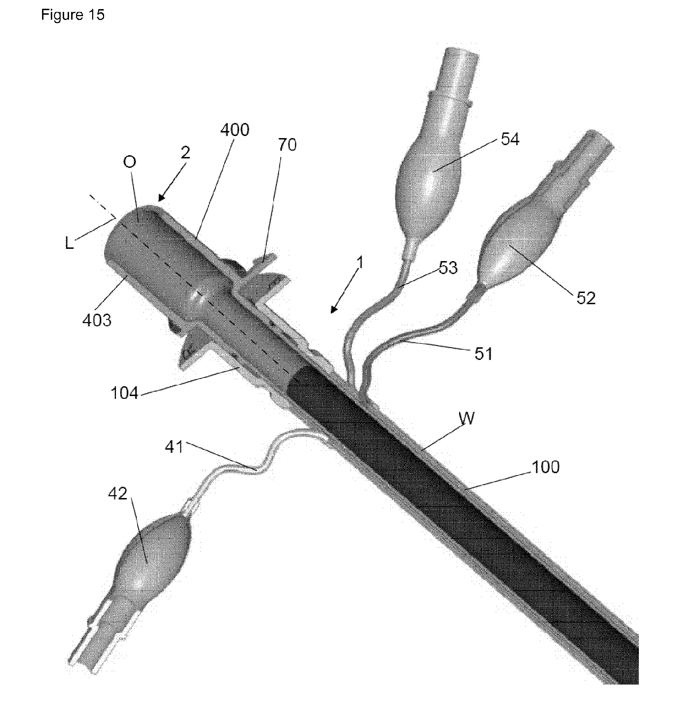

Now, the second opening 103 can be closed by means of said further closure element 700 as shown in Fig. 13 by inflating the further closure element (inflatable balloon) 700 with a medium (gas or liquid) via a conduit 53 (inflation line) connected e.g. to a pilot balloon 54 (cf. Fig. 15). The inflated balloon 700 then blocks the endobronchial portion 200 thus closing the second opening 103 so that no inflation gas G can be discharged out of outlet O'. In Fig. 14 the inflatable balloon 700 is in a non-inflated state leaving the second opening 103 open so that inflation gas G can pass through the endobronchial portion 100 and can then be discharged through outlet O'.

Further, as shown in Fig. 15, the first end section 403 of the closure element may comprises an indicating means 70 that protrudes from the first end section 403 of the inner tube 400, which indicating means 70 preferably points in the direction of the aperture 401 of the closure element 400, so that the respective physician or medical personnel can see whether the first opening 102 is opened or closed.

Further, as shown in Figs. 7 and 8, the tube 1 may comprise an arresting means 60 (not shown in Fig. 15) for arresting the closure element (inner tube) 400 in its first and second position with respect to the surrounding endotracheal portion 100 of the tube 1.

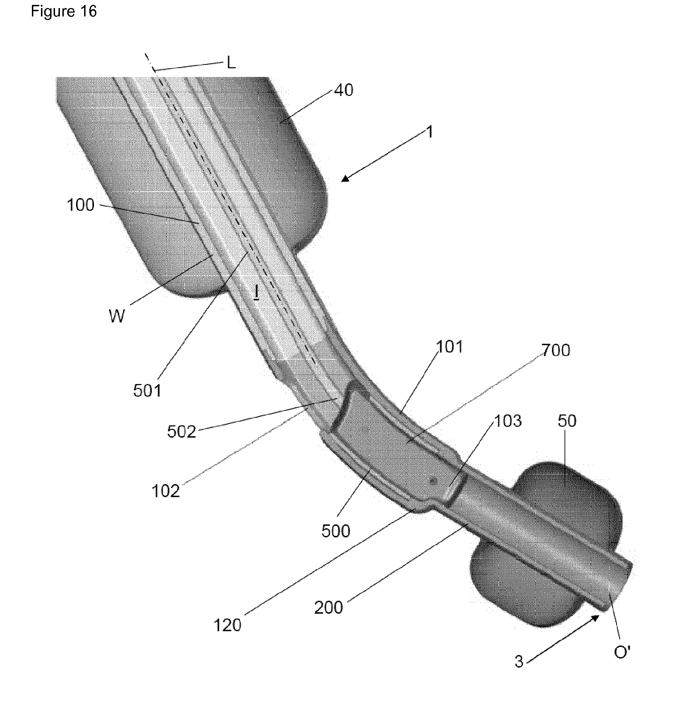

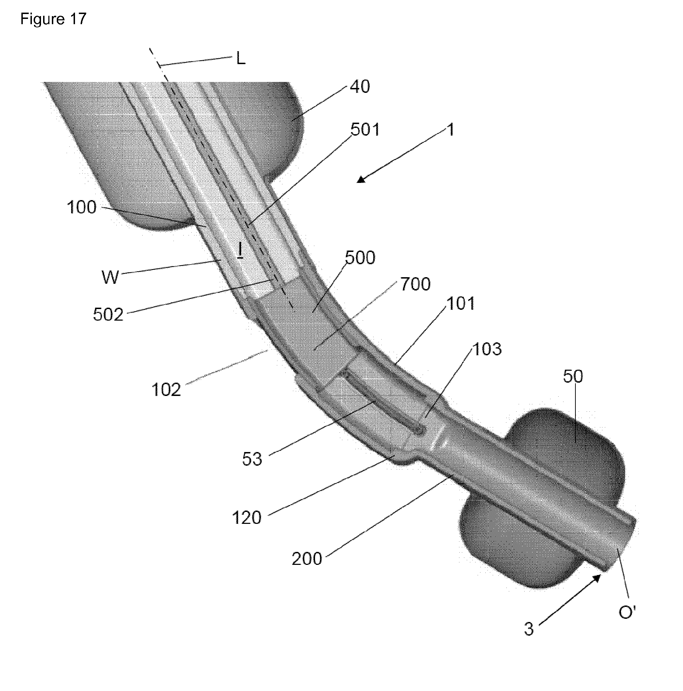

Figs. 16 to 18 show an alternative way of closing and opening the first and second opening 102, 103. Here, the closure element 500 is formed as a sliding element 500 being arranged in said lumen or cavity l of the tube 1 such that it can be slid or displaced along the longitudinal axis L of the endotracheal portion 100 between a first position, in which the closure element 500 is pushed past the first opening 102 along said axis L thus leaving the first opening 102 uncovered and therefore open (cf. Fig. 16), into a second position, in which said sliding element 500 covers the first opening 102 from the inside and thus closes it (cf. Fig. 17). Thus, in the first position of the closure element 500 ventilation gas G can be discharged out of the first opening 102, while in the second position of the closure element 500 the first opening 102 is blocked for preventing the passage of ventilation gas G. Particularly, the closure element 500 may have a tubular shape and may be arranged with a positive fit in said lumen or cavity l in the end region 101 of the endotracheal portion 100 of the tube 1.

In order to move the closure element 500 between said positions, the latter is connected to an end region 502 of an elongated member 501 extending inside said lumen or cavity l of the tube 1 along the longitudinal axis L of the endotracheal portion 100. Said elongated member 501 is connected to a handle (e.g. slider) 504 at a further end region 503 (opposing end region 502 of said member 501) of the elongated member 501, which handle 504 is arranged in the region of the proximal end 2 of the tube 1.

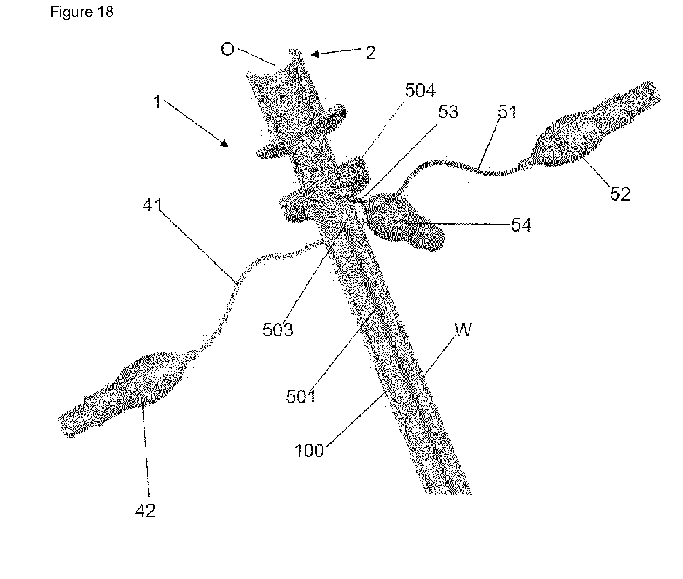

The handle 504 is designed to be slid back and forth along the longitudinal axis L of the endotracheal portion 100 taking along the elongated member 501 and thus the closure element 500 connected to the elongated member 501. In this way, the closure element (sliding element) 500 can be displaced back and forth between the first and second position manually by means of said handle 504 (cf. Fig. 18).

Preferably, for opening and closing the second opening 103, the tube 1 comprises a further closure element 700 in the form of an inflatable balloon that is designed to close or block the second opening 103 for preventing the passage of ventilation gas G in an inflated state, and to leave the second opening 103 open in a non-inflated state so that ventilation gas G can pass the second opening 103. Said further closure element 700 is fastened to the closure element 500 and preferably encompassed by the closure element 500, such that the further closure element 700 can be displaced together with the closure element 500,

As shown in Fig. 16, the further closure element 700 is preferably inflated in the first position of the closure element 500 in which the closure element 700 is positioned beyond the first opening 102 along the longitudinal axis L of the endotracheal portion 100 such that the inflated balloon 700 blocks the second opening 103 but leaves open the first opening 102. Thus ventilation gas G can then still be discharged through the first opening 102 out of the endotracheal portion 100 of the tube 1.

As before, the inflatable balloon 700 is designed to be inflated with a gaseous or liquid medium via a conduit 53 (inflation line) by means of a pilot balloon 54, for instance (cf. Fig. 18).

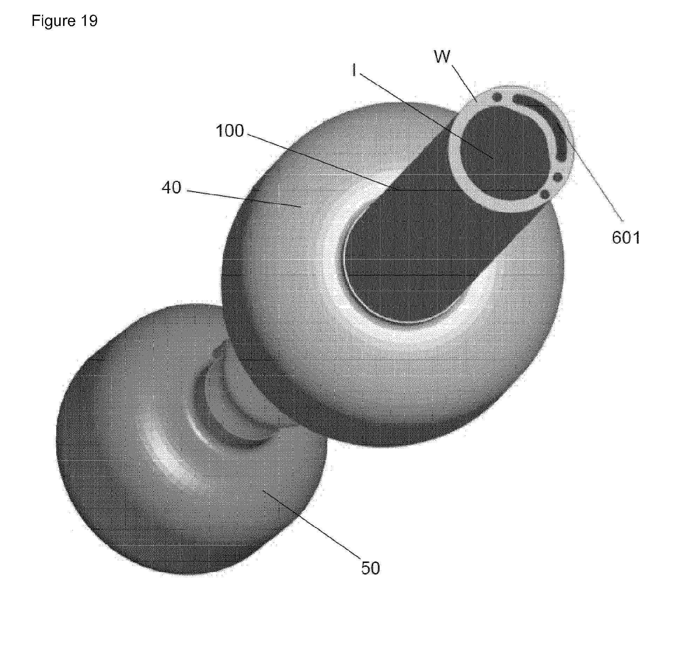

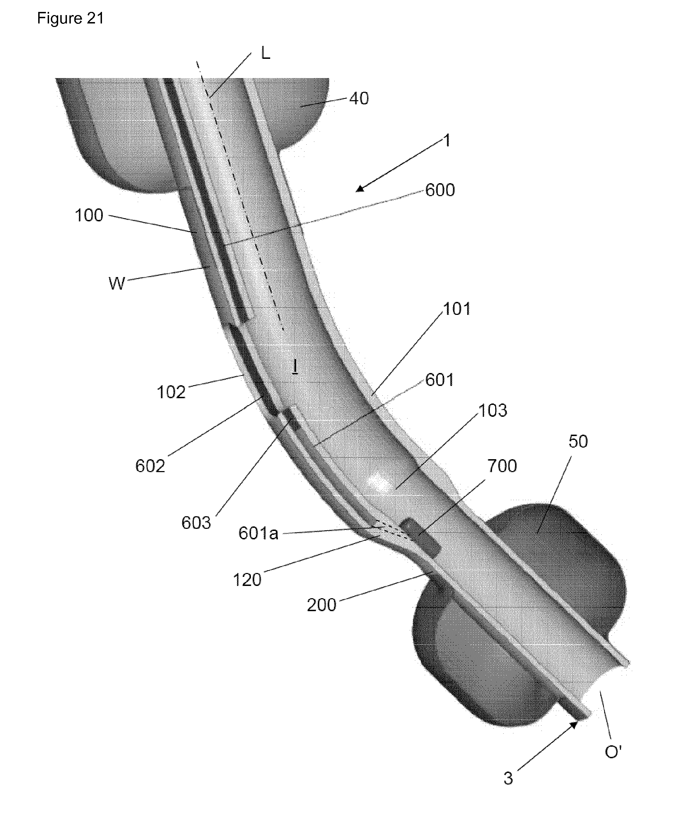

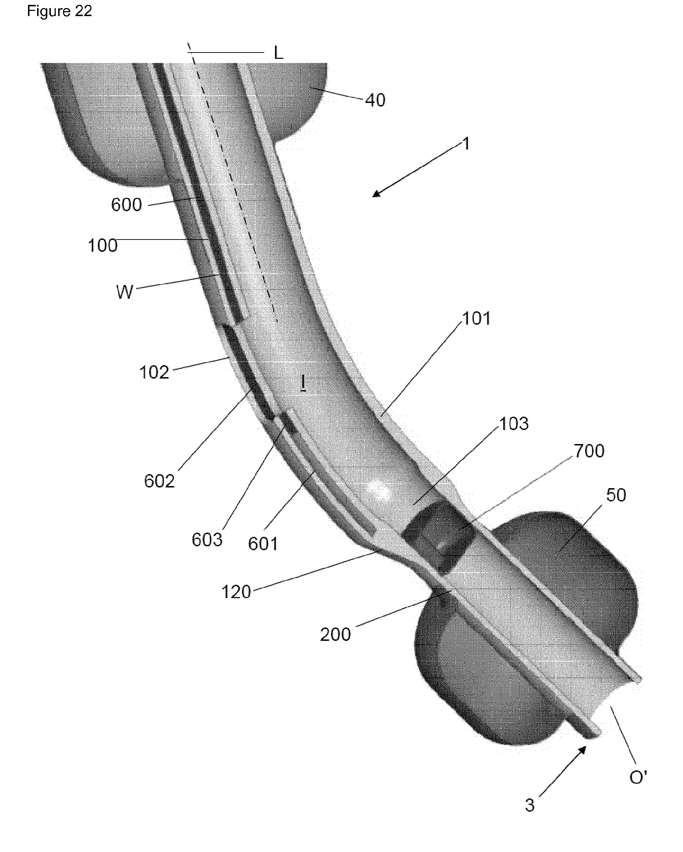

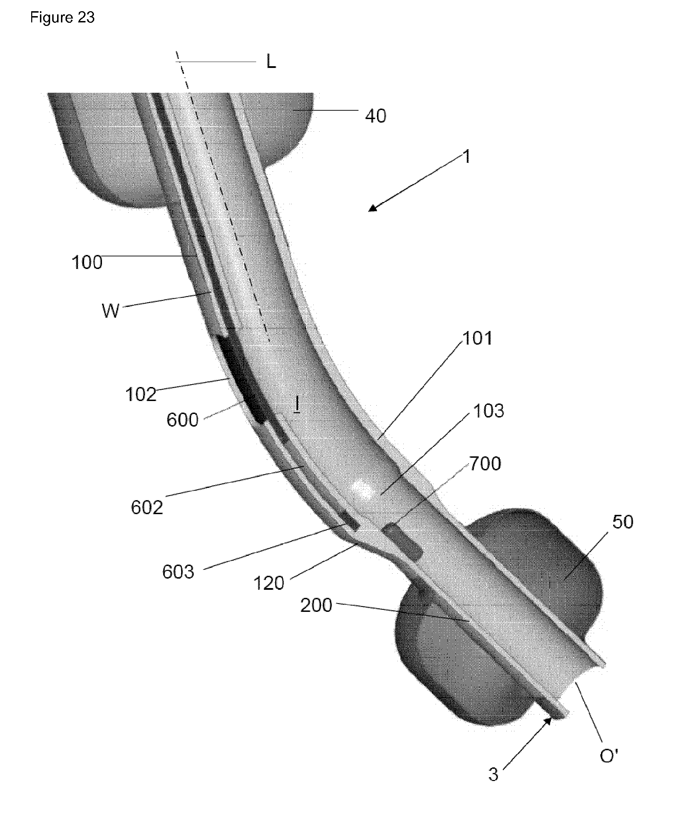

In yet another preferred embodiment of the present invention shown in Figs. 19 to 24, the closure element 600 is formed as an elongated sliding element 600 extending along the longitudinal axis L of the endotracheal portion 100 of the tube 1, which sliding element 600 is housed (particularly with a positive fit) in a separate cavity 601 (cf. Fig. 19) and can be displaced along the longitudinal axis L between a first position (shown in Figs. 21 and 22) and a second position (shown in Fig. 23). Said separate cavity 601 is formed in a wall W of the endotracheal portion 100 and extends longitudinally along the longitudinal axis L of the endotracheal portion 100.

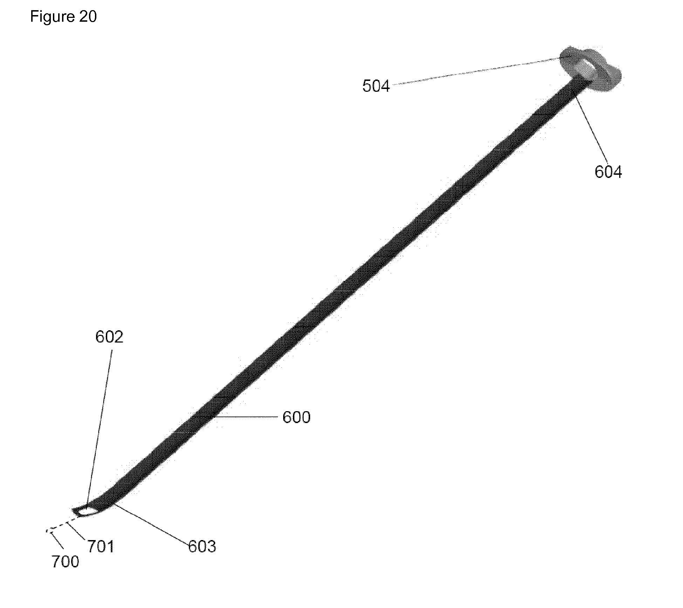

As shown in Fig. 20 the closure element 600 comprises an aperture 602 in a first end region 603, which, in the first position of the closure element 600 is aligned with the first opening 102 such that ventilation gas G can be discharged out of the endotracheal portion 100 through the first opening 102. In the second position, said aperture 602 is arranged away from the first opening 102, namely beyond the first opening 102 along the longitudinal axis L (towards the endobronchial portion 200), so that the first opening 102 is covered by a continuous portion of the closure element 600 and is thus closed (i.e. blocked for preventing the passage of ventilation gas G).

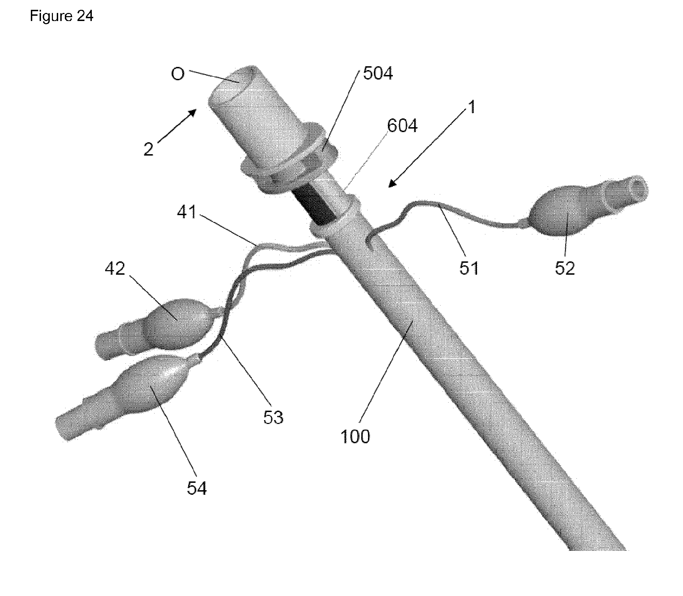

As before, a handle (e.g. slider) 504 is connected to the sliding element 600 at an opposing second end region 604 of the sliding element 600, which handle 504 is arranged in the region of the proximal end 2 of the tube 1 and is designed to be slid back and forth along the longitudinal axis L of the endotracheal portion 100, whereby it takes along the sliding element 600 such that the sliding element (closure element) 600 can be displaced back and forth between the first and second position manually by means of said handle 504 (cf. Fig. 24).

As shown in Figs. 21 to 23, for closing the second opening 103, a further closure element 700 in the form of an inflatable balloon 700 is provided in the lumen or cavity l in the endobronchial portion 200 of the tube 1 adjacent to the second opening 103. Preferably, the inflatable balloon 700 is fastened to an inner side of the endobronchial portion 200 of the tube 1. In an inflated state (cf. Fig. 22), said balloon 700 closes the second opening 103 by blocking the endobronchial portion 200, so that no ventilation gas G can be discharged through outlet O' of the tube 1 or endobronchial portion 200, wherein in an non-inflated state (shown in Figs. 21 and 23) the inflatable balloon leaves the second opening 103 open (i.e. the endobronchial portion 200 is not blocked and allows for passage of ventilation gas G such that the latter can be discharged through outlet O' of the tube 1).

As before, the inflatable balloon 700 is designed to be inflated with a gaseous or liquid medium via a conduit 53 (inflation line) by means of a pilot balloon 54, for instance (cf. Fig. 24).

Alternatively, the inflatable balloon 700 is not attached to said inner side of the endobronchial portion 200, but to the sliding element 600 as indicated in Fig. 20 by the dashed lines. In this case, the further closure element in the form of the inflatable balloon 700 is preferably fastened to the sliding element 600 at a first end or end region 603 of the sliding element 600, particularly via a conduit (e.g. inflation line) 701 of the inflatable balloon 700 that preferably extends along or inside the sliding element 600, wherein said inflation line 701 may protrude from the sliding element 600 through a channel 601a (indicated with dashed lines in Fig. 21) that connects said cavity 601 with said single lumen l of the tube 1 in the region of the endobronchial portion 200 of the tube 1 so that the inflatable balloon 700 connected to said inflation line 701 is positioned in the endobronchial portion 200 of the tube 1 in order to close the second opening 103 when inflated via said inflation line 701.

## Claims

1. Tube for endobronchial intubation, comprising
- an endotracheal portion (100) being designed to be inserted into the trachea (10), the endotracheal portion (100) being in fluid communication with an inlet (O) at a proximal end (2) of the tube (1) for feeding a ventilation gas (G), particularly air, into the tube (1),
- an end region (101) of the endotracheal portion (100) opposing the proximal end (2), said end region (101) comprising a first opening (102) for discharging said ventilation gas (G) in one of the main bronchi (11) and a second opening (103) for discharging ventilation gas (G) in the other main bronchus (12),
- an endobronchial portion (200) being designed to be inserted into said other main bronchus (12), wherein the endobronchial portion (200) is connected to said end region (101) and is in fluid communication with said second opening (103) such that said ventilation gas (G) can be discharged through the second opening (103) into the endobronchial portion (200), wherein the endobronchial portion (200) further comprises an outlet (O') at a distal end (3) of the tube (1), so that said ventilation gas (G) can be discharged through said outlet (O') into said other main bronchus (12), and
- wherein particularly said endotracheal portion (100) and said endobronchial portion (200) surround a single lumen (I) for the passage of said ventilation gas (G) through the tube (1) from said inlet (O) to said outlet (O'),
**characterized in that,**
the tube (1) comprises a closure element (300; 400; 500; 600) for closing and opening said first and/or second opening (102, 103).

2. Tube according to claim 1, **characterized in that** the closure element (300; 400) is a rotatable closure element (300; 400).

3. Tube according to claim 1 or 2, **characterized in that** the closure element (300) is designed to be rotated between a first and a second position, wherein in the first position, the closure element (300) closes the second opening (103) by covering it and opens the first opening (102) by uncovering it, and wherein in the second position, the closure element (300) closes the first opening (102) by covering it and opens the second opening (103) by uncovering it.

4. Tube according to claim 3, **characterized in that** the closure element (300) is further designed to be rotated between the first and the second position via a third position, wherein in the third position the closure element (300) opens the first and the second opening (102, 103) at the same time by uncovering them.

5. Tube according to claim 3 or 4, **characterized in that** the closure element (300) comprises an aperture (301), wherein in the first position of the closure element (300) said aperture (301) lies on top of the first opening (102), and wherein in the second position (103) of the closure element (300) said aperture (301) lies on top of the second opening (103), and wherein particularly in said third position the aperture (301) lies on top of both the first and the second opening (102, 103).

6. Tube according to claim 3 or 4, **characterized in that** the closure element (300) comprises a covering region (320) wherein in the first position of the closure element (300) the covering region (320) covers the second opening (103) for blocking passage of ventilation gas (G) through the second opening (103) and leaves open the first opening (102), and wherein in the second position of the closure element (300) said covering region (320) covers the first opening (102) for blocking passage of ventilation gas (G) through the first opening (102), and leaves open the second opening (103), and wherein particularly in said third position the covering region (320) leaves open both the first and the second opening (102, 103), so that ventilation gas (G) can flow through both openings (102, 103).

7. Tube according to claim 5, **characterized in that** the closure element (300) comprises a tapered region (302) or is formed as a tapered region (302), in which tapered region (302) said aperture (301) is formed.

8. Tube according to claim 1 or 2, **characterized in that** the closure element (400) is designed to close and open said first opening (102), wherein the closure element (400) is designed to be rotated between a first and a second position, wherein in the first position, the closure element (400) opens the first opening (102) by uncovering it, and wherein in the second position, the closure element (400) closes the first opening (102) by covering it.

9. Tube according to claim 8, **characterized in that**
- the closure element (400) comprises an aperture (401), wherein in the first position of the closure element (400) said aperture (401) lies on top of the first opening (102), and wherein in the second position of the closure element (400) said aperture (401) lies on top of a wall region (W') of said end region (101), or
- the closure element (400) comprises a covering region wherein in the first position of the closure element (400) the covering region lies on top of a wall region (W') of said end portion (101) and leaves the first opening (102) open, and wherein in the second position of the closure element (400) said covering region covers the first opening (102) for blocking passage of ventilation gas (G) through the first opening (102).

10. Tube according to one of the claims 8 to 9, **characterized in that** the closure element (400) comprises a cylindrical region (402) or is formed as a cylindrical region (402), in which cylindrical region (402) said aperture (401) is formed.

11. Tube according to one of the preceding claims, **characterized in that** the endotracheal portion (100) of the tube (1) extends along a longitudinal axis (L).

12. Tube according to claim 11, **characterized in that** the closure element (300; 400), particularly the tapered region (302) or the cylindrical region (402), is designed to rotate about the longitudinal axis (L) of the endotracheal portion (100).

13. Tube according to one of the preceding claims, **characterized in that** the closure element (300; 400), particularly the tapered region (302) or the cylindrical region (402), is designed to butt with an outer side (302a; 402a) against an inner side (101 a) of the end region (101) of the endotracheal portion (100) of the tube (1), particularly in a form fitting manner, wherein particularly said inner side (101 a) faces said lumen (I) of the tube (1).

14. Tube according to one of the preceding claims, **characterized in that** the closure element (300; 400) is formed as an inner tube inserted into the endotracheal portion (100), particularly in a form fitting manner, wherein said inner tube (300; 400) protrudes out of the endotracheal portion (100) with a first end section (303), and wherein particularly said tapered region (302) or said cylindrical region (402) forms an opposing second end section (302; 402) of said inner tube (300; 400).

15. Tube according to claim 14, **characterized in that** said first end section (303; 403) of the closure element (300; 400) is designed to be rotated manually in order to rotate the closure element (300; 400).

16. Tube according to claim 1, **characterized in that** the closure element (500; 600) is designed to open and close said first opening (102), wherein the closure element (500; 600) is designed as a sliding element (500; 600) that is designed to be displaced between a first and a second position, wherein in the first position the closure element (500; 600) leaves the first opening (102) open, and wherein in the second position the closure element (500; 600) covers the first opening (102), wherein particularly the closure element (500) is arranged in said single lumen (I) or wherein particularly the closure element (600) is arranged in a separate cavity (601) formed in a wall (W) of the endotracheal portion (100).

17. Tube according to one of the claims 8 to 16, **characterized in that** the tube (1) comprises a further closure element (700) in the form of an inflatable balloon (700) that is designed to close the second opening (103) in an inflated state, and to leave the second opening (103) open in a non-inflated state, wherein particularly the further closure element (700) is arranged in a region of said single lumen (I) delimited by the endobronchial portion (200) or wherein particularly the further closure element (700) is fastened to the closure element (500; 400; 600), such that particularly the further closure element (700) can be displaced together with the closure element (400; 500; 600).
